# EUROPÄISCHE PATENTANMELDUNG

(11) **EP 2 062 542 A1**
(43) Veröffentlichungstag der Anmeldung: **27.05.2009**
(21) Anmeldenummer: 07405331.5
(22) Anmeldetag: 23.11.2007
(51) Int. Cl.: A61B 17/00, A61B 17/08, A61F 13/00

(54) **Vorrichtung zum Wiederverschliessen eines druckbeaufschlagten komprimierbaren elastischen Gefässes**

(71) Anmelder: INOVA MEDICAL AG, 6330 Cham (CH)
(72) Erfinder: Harren, Ernst Diethelm, 6312 Steinhausen (CH); Stanek, Jan, 53705 Chrudim (CZ)
(74) Vertreter: EGLI-EUROPEAN PATENT ATTORNEYS

(57) **Zusammenfassung**

Eine Vorrichtung zum Wiederverschliessen eines druckbeaufschlagbaren, komprimierbaren elastischen Gefässes nach einer Punktion desselben, hat ein zylinderförmiges oder tropfenförmiges Verschlussteil (1) aus einem leicht durchdringbaren ersten Material mit hoher Rückstellkraft, z.B. aus Silikon. Die Vorrichtung funktioniert unter Verwendung des Mediums, das den Druck in dem Gefäss erzeugt. Die Vorrichtung hat auch einen Verschlussteilträger (2) oder Teile des Verschlussteilträgers (2) aus einem flexiblen und dehnbaren zweiten Material, sowie ein Druckwandteil (3) aus einem dünnen, flexiblen und dehnbaren dritten Material, das mit dem zweiten Material des Verschlussteilträgers (2) oder Teile des Verschlussteilträgers (2) verbunden ist. Zwischen dem Verschlussteil (1) und dem Druckwandteil (3) ist eine Druckkammer (4) für das Medium ausgebildet oder ausbildbar. Dabei ist das Verschlussteil (1) unlösbar in eine Ausnehmung (5) im Verschlussteilträger (2) eingefügt.

## Beschreibung

Die Erfindung betrifft eine Vorrichtung nach Patentanspruch 1 zum Wiederverschliessen eines druckbeaufschlagbaren, komprimierbaren elastischen Gefässes, nach einer Punktion desselben, mittels des Mediums, welches den Druck in dem Gefäss erzeugt.

Die vorliegende Erfindung ist sowohl für Anwendungen am menschlichen oder tierischen Körper wie auch für Anwendungen ausserhalb des menschlichen oder tierischen Körpers vorgesehen.

Bei der Anwendung am menschlichen oder tierischen Körper dient die vorliegende Erfindung zum Verschliessen eines eine Punktionsöffnung aufweisenden arteriellen oder venösen Blutgefässes im menschlichen oder tierischen Körper mittels Eigenblut. In diesem Fall ist Blut das Medium, welches den Druck in dem Gefäss erzeugt. Weitere Anwendungen sind auch bei mit Arterien kurzgeschlossenen Venen, bei Shunts, bei Prothesen und dergleichen.

Bei der Anwendung ausserhalb des menschlichen oder tierischen Körpers kann das druckerzeugende Medium natürlich auch eine andere Flüssigkeit sein. So ist es denkbar, dass beispielsweise für Probenahmen flüssigkeitsgefüllte Beutel oder sonstige elastische Gefässe punktiert werden müssen, ohne dass deren Gebrauchstüchtigkeit wesentlich beeinträchtigt ist.

In beiden Fällen geht es darum, nach einer Punktierung entweder eine Blutung aus dem Gefäss zu stillen oder aber das weitere Auslaufen einer Flüssigkeit aus einem Gefäss zu stoppen.

Die Erfindung macht dabei Gebrauch von den (bei Punktionsverschlüssen) bereits bekannten Prinzipien, im Bereich der Punktionsöffnung eine am Körper befestigbare Druckkammer anzubringen, die sich durch einströmendes Blut füllt und in der sich zum Zweck der Blutstillung ein Druckgleichgewicht aufbaut. Zum Zweck der Selbst-Wiederverschliessbarkeit muss dabei im Bereich der Druckkammer auch ein durchstechbares Verschlussteil aus einem Material mit hoher Rückstellkraft vorhanden sein. Die hohe Rückstellkraft gewährleistet, dass sich der Durchstichkanal nach dem Rückzug des Punktierungsinstrumentes wieder verschliesst.

Aus der EP-0 776 179 (siehe auch WO-1996/05774) ist ein Punktionsverschluss zum Verschliessen eines eine Punktionsöffnung aufweisenden Blutgefässes bekannt. Zwei Ausführungsformen sind vorgesehen.

Eine erste Ausführungsform hat ein durchstechbares, aber nicht selbstschliessendes Halteband mit einem vorbereiteten, nachträglich auf das Punktierungsloch klebbaren Verschlusselement. Eine Druckkammer befindet sich unter der zum Einstich vorgesehenen Stelle des Haltebandes.

Eine zweite Ausführungsform hat ein Halteband mit einer für den Einstich vorgesehenen Aussparung. Auch hier gibt es eine Druckkammer, die sich unter der zum Einstich vorgesehenen Stelle des Haltebandes befindet, in diesem Fall unter der Aussparung. Ein vorbereitetes Verschlusselement mit einem Druckpolster ist nach dem Einstich und dem Rückzug der Nadel in die Aussparung einsetzbar. Bei dieser Lösung sind weder das Halteband noch das vorbereitete Verschlusselement zum Durchstechen vorgesehen. Das vorbereitete Verschlusselement hat ein zylinderförmiges Druckpolster, das in die Aussparung passt. Das zylinderförmige Druckpolster ist nicht selbstverschliessend, aber es ist (wie bei der ersten Ausführungsform) ebenfalls mittels einer lösbaren Klebeverbindung auf das Halteband aufgesetzt.

Ein Hauptnachteil diese Punktionsverschlusses besteht denn auch darin, dass die Anwendung grosses Geschick und auch Schnelligkeit erfordert, weil ausströmendes Blut sonst sehr schnell die Haftfähigkeit des vorbereiteten Verschlusselementes beeinträchtigt. Gefässverschlüsse mit (nach dem Einstich und dem Zurückziehen der Nadel) selbstverschliessendem Verschlussteil stellen deshalb klar eine vorteilhafte Weiterentwicklung dieses Punktions-verschlusses dar.

Aus der EP-0 955 901 (siehe auch WO-1998/22027) ist ein dichter Punktionsverschluss bekannt. Hier ist ein selbstverschliessendes Verschlussteil klar vorhanden. Auch hier sind zwei Ausführungsformen vorgesehen.

In einer ersten Ausführungsform ist ein zylinderförmiges selbstverschliessendes Verschlusselement aus Silikon auf eine Haltewand aufgeklebt. Eine Druckkammer befindet sich unter der zum Einstich vorgesehenen Stelle der Haltewand bzw. unter dem selbstverschliessenden Verschlusselement. Die Haltewand ist aus einer Polyetherurethan- Polyether- oder Polypropylenfolie.

In einer zweiten Ausführungsform ist die Haltewand selber selbstverschliessend ausgebildet. Vorgesehene Materialien dazu sind u.a. Kautschuk, Latex oder Silikon. Die Druckkammer befindet sich auch hier unterhalb der zum Einstich vorgesehenen Stelle der Haltewand.
Nachteile ergeben sich jedoch, weil jeweils mehrerer Teile, so etwa die Haltewand und das zylinderförmige Verschlusselement, separat hergestellt, positioniert und miteinander verbunden werden müssen, was einen relativ hohen Herstellungsaufwand darstellt. Ist das zylinderförmige Verschlusselement aus Silikon, so ist vorgesehen, für die Verbindung desselben mit der Haltewand einen speziellen Kleber einzusetzen, weil gewöhnliche Kleber für Silikon nicht einsetzbar sind. Im Falle der zweiten Ausführungsform erweist es sich trotz des einfachen Aufbaus als nachteilig, dass im Falle der ausschliesslichen Verwendung von Silikon für die Haltewand gerade wegen der extrem hohen Elastizität und Rückstellkraft dieses Materials die notwendige mechanische Dehnfestigkeit in diesem Bereich oftmals nur schwer erreichbar ist.

Aus der WO-2006/042431-A1 ist ein weiterer externer Gefässverschluss bekannt bei dem ein zylinderförmiges selbstverschliessendes Verschlussteil klar vorhanden ist.

Dieser Gefässverschluss hat eine mit Überdruck beaufschlagbare, im Bereich der Punktionsöffnung am Körper befestigbare Druckkammer, die in ihrem vom Körper abgewandten Bereich ein einstückig ausgebildetes Verschlussteil aus Silikon mit einem integrierten Verschlusselement aufweist. Die Druckkammer hat in ihrem dem Körper zugewandten Bereich ein einstückig ausgebildetes Druckwandteil. Dieser externe Gefässverschluss ist aufklebbar, transparent und von einer Nadel einer Kanüle, einem Katheter oder dergleichen durchstechbar. Das Druckwandteil erstreckt sich mit Ausläufern auf den gesamten auf den Körper aufzuklebenden Bereich des externen Gefässverschlusses, und zwischen dem Druckwandteil und dem Verschlussteil ist mit Ausnahme des Druckkammerbereiches grossflächig eine Kleberschicht angebracht.

Obwohl dieser Gefässverschluss gegenüber demjenigen gemäss WO-1998/22027 den Vorteil einfacherer Herstellbarkeit hat, besteht ein Nachteil darin, dass - infolge der einstückigen Ausbildung des Verschlussteils - im Bereich der Ausläufer ein bei der Massenherstellung nicht unbeträchtlich hoher Anteil an für medizinische Zwecke geeignetem Silikon verwendet werden muss, der mit Hinblick auf die in diesem Teilbereich erforderlichen Materialeigenschaften wohl ebensogut durch ein anderes, billigeres Material ersetzt werden könnte. Zudem ist ein derartiger Gefässverschluss - wiederum wegen der ausschliesslichen Verwendung von für medizinische Zwecke geeignetem Silikon für das einstückige Verschlussteil - für Anwendungen ausserhalb des menschlichen oder tierischen Körpers, wo die medizinische Verträglichkeit oder die Hautverträglichkeit meist nicht mehr an erster Stelle stehen, meist auch zu teuer.

Aufgabe der Erfindung ist es, eine weiter verbesserte Vorrichtung zum Wiederverschliessen eines druckbeaufschlagbaren, komprimierbaren elastischen Gefässes anzugeben. Die erfindungsgemässe Vorrichtung soll sowohl für den Gebrauch am menschlichen oder tierischen Körper wie auch für den Gebrauch ausserhalb des menschlichen oder tierischen Körpers verwendbar sein.

Diese Aufgabe wird durch die Merkmale des Patentanspruchs 1 gelöst.

Die Lösung besteht darin, dass bei einem gattungsgemässen Gefässverschluss ein erhabenes, vorzugsweises zylinderförmiges, tropfenförmiges oder halbkugelförmiges Verschlussteil aus einem leicht durchdringbaren Material mit hoher Rückstellkraft unlösbar in eine Ausnehmung in einem Verschlussteilträger eingefügt ist.

Silikon ist bekanntlich eines der am häufigsten verwendeten Materialien, die diese Eigenschaften aufweisen. Silikon, insbesondere medizinisches Silikon, ist (von Kanülen und dergleichen) leicht durchdringbar und hat eine sehr hohe Rückstellkraft. Es hat deshalb die erwünschte selbsttätige Wiederverschliesseigenschaft und ist zudem auch aus medizinischer Sicht bestens geeignet. Es hat sich aber auch gezeigt, dass Silikon mit anderen Materialien nicht leicht und nicht ohne weiteres dauerhaft verbindbar ist. Um Klebeverbindungen mit Silikon herzustellen, braucht es spezielle Kleber, weil gängige Klebstoffe nicht haften oder aus anderen Gründen ungeeignet sind. Einer der Hauptgründe ist hier natürlich wiederum die medizinische Verträglichkeit der Klebstoffe, die bei der Anwendung an Mensch und Tier natürlich immer beachtet werden muss.

Es hat sich nun aber gezeigt, dass Einspritzverfahren von Silikon in Formen aus Materialien bestimmter thermoplastischer Elastomere oder technischer Silikone eine chemische Anbindung ergeben, die ausgezeichnete Festigkeitswerte hat und deshalb als unlösbar bezeichnet werden kann.

Dies hat den Vorteil, dass sich in erfindungsgemässer Weise zylinder- oder tropfenförmige Verschlussteile aus Silikon bei automatisierter industrieller Produktion leicht in Ausnehmungen in Verschlussteilträger aus thermoplastischen Elastomeren oder aus technischen Silikonen einspritzen lassen. Somit kann die industrielle Fertigung in grossen Stückzahlen rationalisiert und beschleunigt werden.

Mit dem gleichen Verfahren können deshalb auch prinzipiell gleichartig aufgebaute Gefässverschlüsse hergestellt werden, die auch ausserhalb des menschlichen oder tierischen Körpers an geeigneten Gefässen anwendbar sind. Die Unterschiede bestehen hauptsächlich darin, dass bei medizinischen Anwendungen an Mensch und Tier auf dem Druckwandteil ein hautfreundlicher Haftkleber angebracht sein muss, während bei einer nicht-medizinischen Anwendung meist ein gewöhnlicher Haftkleber genügt. Oft bestehen bei nicht-medizinischen Anwendungen deshalb auch nicht die gleichen Anforderungen an das Material mit hoher Rückstellkraft, das für das Verschlussteil verwendet wird (so könnten dann z.B. auch nicht-medizinische Silikone geeignet sein). Zudem kann bei nicht-medizinischen Anwendungen davon ausgegangen werden, dass eine Druckkammer zwar vorhanden sein kann, aber nicht muss. Bei all dem muss aber auch noch bedacht werden, dass es auch Anwendungen für derartige Vorrichtungen ausserhalb des menschlichen oder tierischen Körpers gibt, bei denen unter Umständen dennoch - mindestens bezüglich der Materialanforderungen - medizinische Kriterien zu erfüllen sind; so etwa bei der Punktierung von Infusionsbeuteln. Auch hier dürfen bei einer Punktierung keine Kleberpartikel ins Innere des Beutels gelangen.

Weitere Vorteile erreicht man, wenn das Verschlussteil transparent ist und verschiedenartig eingefärbte oder verschiedenartig getönte oder verschiedenartig oberflächig behandelte Schichten oder Teilbereiche oder verschiedenartige Geometrien zum Zweck der Vorgabe eines optimalen Einstichwinkels aufweist. Auf einfache Weise kann so dafür gesorgt werden, dass dem Personal, das die Punktierung durchzuführen hat, eine visuelle Hilfe zur Durchführung des Punktierungsvorganges zur Verfügung steht.

Weiterhin kann durch geeignete Formgebung des Verschlussteils und des Verschlussteilträgers erreicht werden, dass die Verbindungsstelle zwischen Druckwandteil und Verschlussteilträger geringeren Aufreisskräften ausgesetzt ist. Es kann im Falle bestimmter Materialkombinationen vorteilhaft sein, die Verbindungsstelle zwischen dem Druckwandteil und dem Verschlussteilträger von Aufreisskräften zu entlasten. Aufreisskräfte könnten sonst nämlich dazu führen, dass die Verbindung zwischen dem Druckwandteil und Verschlussteilträger vom Rand her (der ja mit dem druckerzeugenden Medium in Kontakt kommt), allmählich aufgerissen oder geschwächt wird. Eine solche Entlastung kann erreicht werden, wenn am Verschlussteil oder am Verschlussteilträger Hinterschnitte oder hinterschnittartige Einformungen zum Zweck der Ausbildung von Gegendruckkammern vorhanden sind. Eine Entlastung kann aber auch erreicht werden, wenn am Verschlussteil oder am Verschlussteilträger Dichtlippen oder dichtlippenartige Ausformungen angebracht sind. Die Grundidee derartiger Entlastungsmassnahmen besteht darin, die bei der Ausbildung der Druckkammer sich ausbildenden Kräfte in einer Weise oder aus einer Richtung wirksam werden zu lassen, dass die Verbindungsfläche zwischen dem Druckwandteil und dem Verschlussteilträger eher oder wenigstens verstärkt zusammengepresst als auseinandergerissen wird. Mit den Dichtlippen erreicht man die Entlastung eher durch Abdeckung der einreissgefährdeten Stellen.

Im folgenden wird anhand von Zeichnungen eine mögliche Ausführungsform der vorliegenden Erfindung näher erläutert. Dabei zeigt
- Fig. 1, 1a: einen Querschnitt durch eine Vorrichtung zum Wiederverschliessen eines druckbeaufschlagbaren, komprimierbaren elastischen Gefässes,
- Fig. 2: einen vergrösserten Ausschnitt des Querschnittes gemäss Fig. 1,
- Fig. 3: eine Draufsicht auf eine Vorrichtung zum Wieder- verschliessen eines druckbeaufschlagbaren, kompri- mierbaren elastischen Gefässes gemäss Fig. 1,
- Fig. 4: eine Seitenansicht eines Verschlussteiles für eine Vorrichtung gemäss Fig. 1,
- Fig. 5a,b eine: schematische Darstellung eines Hinterschnit- tes, entweder im Verschlussteilträger (Fig. 5a) oder im Verschlussteil (Fig. 5b), und
- Fig. 6a,b: eine schematische Darstellung einer Dichtlippe, entweder am Verschlussteilträger (Fig. 6a) oder am Verschlussteil (Fig. 6b).

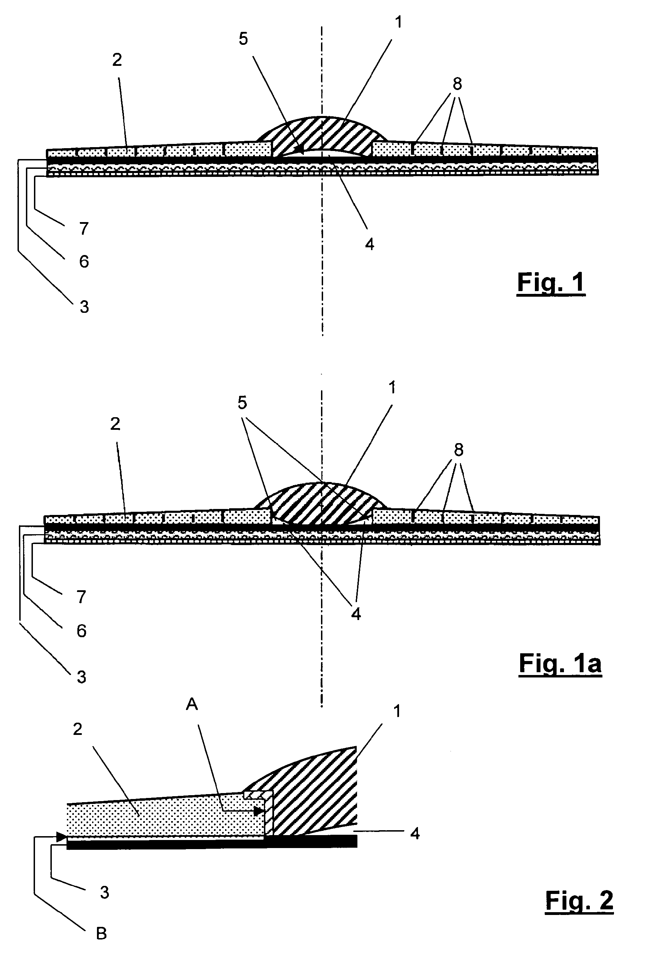

Die Fig. 1 und 1a zeigt einen Querschnitt durch eine Vorrichtung zum Wiederverschliessen eines druckbeaufschlagbaren, komprimierbaren elastischen Gefässes. Das Gefäss selber ist hier nicht dargestellt. Die Vorrichtung dient zum Wiederverschliessen des Gefässes nach einer Punktion desselben und macht dazu Gebrauch von dem Medium, das den Druck in dem Gefäss erzeugt. Die Vorrichtung gemäss Fig. 1 und Fig. 1a ist speziell geeignet für den Gebrauch am menschlichen oder tierischen Körper, sie ist aber beispielsweise auch für den Gebrauch von Probenahmen aus druckbeaufschlagten durchstechbaren Gefässen oder Behältnissen unter Labor-, Praxis- oder Feldbedingungen anwendbar.

Im Fall des Gebrauchs der Vorrichtung am menschlichen oder tierischen Körper ist das druckbeaufschlagbare, komprimierbare elastische Gefäss eine Arterie oder eine Vene und das druckerzeugende Medium ist Blut. In diesem Fall geht es bei der Anwendung um die Blutstillung. Weitere Anwendungen sind auch bei mit Arterien kurzgeschlossenen Venen, bei Shunts, bei Prothesen und dergleichen. Im Fall des Gebrauchs der Vorrichtung ausserhalb des menschlichen oder tierischen Körpers kann das druckbeaufschlagbare, komprimierbare elastische Gefäss beispielsweise ein Infusionsbeutel, eine Probenahmebeutel oder ein ähnliches Behältnis sein und das druckerzeugende Medium kann demzufolge irgendeine andere Flüssigkeit (medizinischer oder technischer Natur) sein. Im letzteren Fall geht es somit um die einfache Entnehmbarkeit von Proben und um das Verhindern des Auslaufens des Mediums aus dem Behältnis nach der Entnahme von Proben.

Die erfindungsgemässe Vorrichtung hat ein zylinderförmiges oder tropfenförmiges Verschlussteil 1 aus einem leicht durchdringbaren ersten Material mit hoher Rückstellkraft. Weiterhin ist ein Verschlussteilträger 2 aus einem flexiblen und dehnbaren zweiten Material und ein Druckwandteil 3 aus einem dünnen, flexiblen und dehnbaren dritten Material vorhanden. Das dritte Material des Druckwandteils 3 ist mit einem gängigen Verbindungsverfahren mit dem zweiten Material des Verschlussteilträgers 2 verbunden. Zwischen dem Verschlussteil 1 und dem Druckwandteil 3 ist eine Druckkammer 4 für das druckerzeugende Medium ausgebildet oder ausbildbar. Das Verschlussteil 1 ist in eine Ausnehmung 5 im Verschlussteilträger 2 unlösbar eingefügt.

Im Ausführungsbeispiel gemäss Fig. 1 ist das Verschlussteil 1 (von unten gesehen) leicht konkav und in Fig. 1a bikonvex und liegt somit nur in den Randbereichen am Druckwandteil 3 an oder gemäss Fig. 1a nur im Mittenbereich. Somit kann man davon sprechen, dass die Druckkammer 4 hier (zumindest teilweise) bereits ausgebildet ist. Das muss aber nicht so sein, das Verschlussteil kann auch überall am Druckwandteil anliegen, so dass man auch von der Ausbildbarkeit einer Druckkammer sprechen kann. Voraussetzung ist in allen drei Fällen lediglich, dass das Verschlussteil und das Druckwandteil nicht miteinander verbunden sind und insbesondere die Spezifikationen des Druckwandteiles gemäss weiter untenstehender Beschreibung im wesentlichen eingehalten sind.

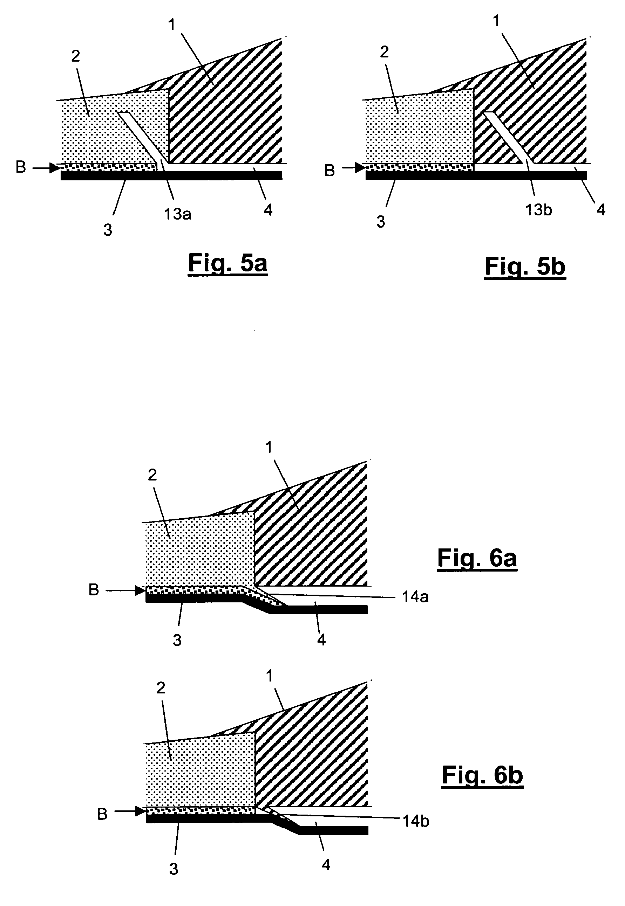

Es ist zudem auch von Vorteil, wenn das Verschlussteil 1 im Bereich der Druckkammer 4 oder im Bereich von Gegendruckkammern (vgl. dazu die Erläuterungen zu Fig. 5) eine ausreichende Rauhigkeit (nicht dargestellt) aufweist. Die Rauhtiefe kann dabei durch Erodieren, Strahlen, Gravieren, Ätzen u.ä. erzeugt werden. Vorzugsweise beträgt die Rauhtiefe grösser 1.6 µm. Diese Massnahme dient dazu, dass das Verschlussteil 1 und der Verschlussteilträger im Bereich der Ausnehmung 5 nicht aneinander haften bleiben. Eine Anhaftung oder Verklebung in diesem Bereich würde dazu führen, dass es beim Herausziehen des Punktierungsinstrumentes zu keiner oder nur zu einer ungenügenden Ausbildung der Druckkammer 4 kommt.

Das Ausführungsbeispiel gemäss Fig. 1 ist, wie oben erwähnt, speziell geeignet für den Gebrauch am menschlichen oder tierischen Körper. Zu diesem Zweck ist auf dem Druckwandteil 3 ein hautfreundlicher Haftkleber 6 angebracht, der seinerseits von einer dünnen, leicht entfernbaren Schutzschicht 7 bedeckt ist. Die Schutzschicht 7 kann dabei als gängige abziehbare Schutzfolie ausgebildet sein.

Das erste Material des Verschlussteils 1 ist vorzugsweise transparentes Silikon. Dabei kann infolge der erwähnten Aufrauhung im Bereich der Druckkamer auch nur eine teilweise Transparenz resultieren. Die für das Verschlussteil 1 erforderlichen Eigenschaften sind damit erfüllt: Silikon ist weich, transparent, leicht durchdringbar, von hoher Rückstellkraft und medizinisch geeignet.

Das zweite Material des Verschlussteilträgers 2 oder eines Teils des Verschlussteiltägers 2 ist vorzugsweise ein thermoplastisches Elastomer (TPE), Polyurethan (PUR), synthetischer Kautschuk (SBC), Ethylenvinylazetat (EVA), Polyolefin (PO), Polyvinylchlorid (PVC) oder ein technisches Silikon. Die Schichtdicke beträgt typischerweise etwa 0.2 - 1.5 mm.

Das dritte Material des Druckwandteils 3 ist vorzugsweise ein transparenter Polyurethan (PUR) -Film mit einer Schichtdicke im Bereich von ca. 25 - 50 µm.

Die allgemeinen Anforderungen an das zweite und dritte Material sind: Weichheit, hohe Flexibilität, Dehnbarkeit, gute Modulierbarkeit (d.h. gute Anpassungsfähigkeit an unterschiedliche Topographien der Haut und anderen Untergründen).

Das erste Material (Silikon) ist mit dem zweiten Material (thermoplastisches Elastomer) mittels eines Einspritzverfahrens verbunden. Dies ergibt die eingangs erwähnte chemische Anbindung (eine Verbindung auf molekularer Ebene), was zu einer praktisch unlösbaren Verbindung zwischen dem Verschlussteil 1 und den Verschlussteilträger 2 führt. Die Region, in der eine derartige chemische Anbindung zwischen den Materialien erfolgt, ist in der Figur 2 mit dem Buchstaben A bezeichnet. Bei einer chemischen Anbindung werden echte chemische Bindungen zwischen funktionellen Gruppen des Thermoplasten und funktionellen Gruppen des Silikons ausgebildet. Die Art der Bindungen hängt natürlich im wesentlichen von den verfügbaren funktionellen Gruppen am Thermoplasten ab. In vielen Fällen mit polaren Thermoplasten dürfte es sich dabei um eine Si-O oder Si-N Bindung(en) handeln.

Das zweite Material (thermoplastisches Elastomer oder technisches Silikon) ist mit dem dritten Material (PUR-Film) wie ebenfalls bereits erwähnt, mit einem gängigen Verbindungsverfahren wie Kleben oder Schweissen verbunden. Die Region, in der eine derart gängige Verbindung zwischen den Materialien erfolgt, ist in der Figur 2 mit dem Buchstaben B bezeichnet.

Weiterhin ist der Verschlussteilträger 2 (und/oder das Druckwandteil 3) in geeigneten Abständen mit Perforationen 8 versehen, um die für Anwendungen am menschlichen oder tierischen Körper notwendige gute Wasserdampfdurchlässigkeit zu erreichen. Es ist aber auch möglich, dass das Druckwandteil 3 selbst ohne Perforationen schon eine genügend hohe Wasserdampfdurchlässigkeit aufweist.

Bezüglich der Funktionsweise der erfindungsgemässen Vorrichtung wird auch auf die eingehenden Beschreibungen im zitierten Stand der Technik verwiesen (insbesondere auf die EP-0 955 901 und die WO-2006/042431-A1). Nach dem Punktieren des Gefässes und nach dem Rückzug der zur Punktierung verwendeten Kanüle füllt sich die Druckkammer 4 mit dem aus dem Punktierungskanal ausströmenden Blut. Wegen der selbstverschliessenden Eigenschaften des Verschlussteiles 1 kann das Blut nicht aus dem Verschlussteil austreten. Die Druckkammer 4 dehnt sich durch das einfliessende Blut aus, und zwar so lange, bis sich ein Druckgleichgewicht ausbildet und die Blutung danach zum Stillstand kommt. Die Rückhaltekräfte des Verschlussteils 1, zusammen mit dem gegen die Punktierungsverletzung des Gefässes gerichteten Druck der nunmehr blutgefüllten Druckkammer 4, bewirken dieses Druckgleichgewicht. Die erfindungsgemässe Vorrichtung wird natürlich so lange am Ort der erfolgten Punktierung belassen, bis die natürlichen Heilkräfte des menschlichen oder tierischen Körpers die Entfernung zulassen, d.h. bis die Punktierungswunde genügend verheilt ist.

Im Falle der Verwendung der Vorrichtung an einem Gefäss ausserhalb des menschlichen oder tierischen Körpers ist natürlich nicht mit einer allmählichen Selbstheilung des punktierten Gefässes zu rechnen, dennoch aber kann eine erfindungsgemäss aufgebaute Vorrichtung in gleicher Weise verwendet werden. Solange die Vorrichtung (nach einer Punktierung) am Einstichort belassen wird, ist das punktierte Gefäss weiter verwendbar, d.h. das im Gefäss vorhandene Medium wird am Auslaufen gehindert.

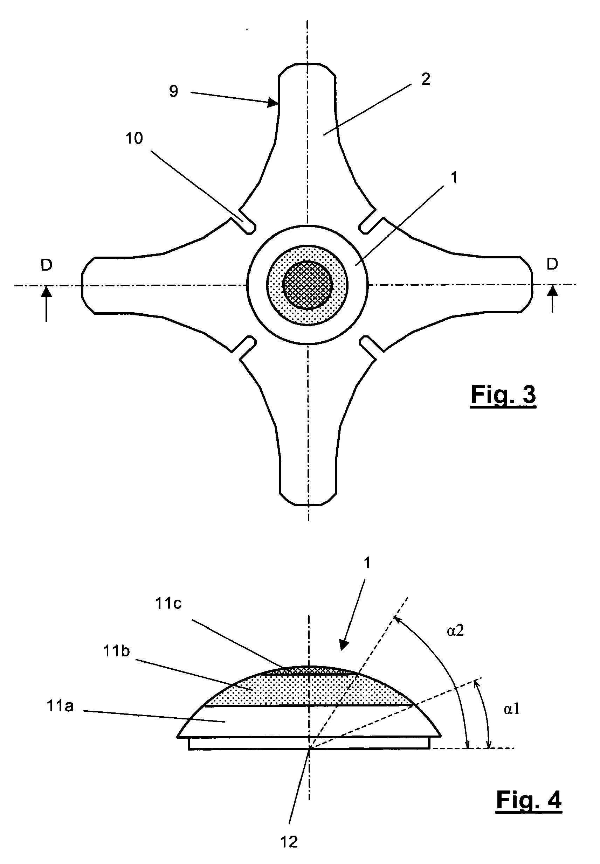

Die Fig. 3 zeigt eine Draufsicht auf eine Vorrichtung zum Wiederverschliessen eines druckbeaufschlagbaren, komprimierbaren elastischen Gefässes gemäss Fig. 1 oder Fig. 1a. In dieser Ausführung hat die Vorrichtung eine im wesentlichen kreuzartige Form, wobei der Verschlussteilträger 2 vier Ausläufer 9 aufweist. Zwischen den Ausläufern 9 hat es Einkerbungen 10. Diese Ausformung des Verschlussteilträgers 2 soll eine möglichst gute Anpassbarkeit an unterschiedlichste topographische Verhältnisse des menschlichen oder tierischen Körpers gewährleisten. Das in der Mitte eingefügte Verschlussteil 1 ist hier (schematisch) in einer Ausführungsform gezeigt, die einem Anwender zusätzlich noch Hilfe zur Wahl des Einstichwinkels bietet (siehe dazu auch Fig. 4)

Die Fig. 4 zeigt eine Seitenansicht eines Verschlussteiles 1 für eine Vorrichtung gemäss Fig. 1 oder Fig. 1a. und 3. Das Verschlussteil 1 weist hier verschiedenartig eingefärbte oder verschiedenartig getönte oder verschiedenartig oberflächig behandelte Schichten oder Teilbereiche 11a,b,c zwecks Vorgabe eines optimalen Einstichwinkels auf. Zum gleichen Zweck könnten aber auch verschiedenartige Geometrien vorgesehen sein. Ein Teilbereich 11a kann beispielsweise völlig transparent und farblos, ein Teilbereich 11b transparent und leicht grau und ein Teilbereich 11c transparent und leicht farbig sein. Dies ergibt eine augenähnliche Struktur, die so gewählt sein kann, dass ein Einstich mit einer Kanüle im Teilbereich 11b und gegen das Zentrum 12 des Verschlussteils stets innerhalb eines vorgegebenen Winkelbereiches zwischen den Winkeln α1 und α2 liegt. Selbstverständlich ist es auch möglich, dass am Verschlussteil 1 vorstehende oder eingelassene Oberflächenstrukturen zum Zweck der Vorgabe eines optimalen Einstichwinkels vorhanden sein können (nicht dargestellt).

Wie eingangs bereits erwähnt, kann weiterhin durch geeignete Formgebung des Verschlussteils 1 und des Verschlussteilträgers 2 erreicht werden, dass die Verbindungsstelle zwischen dem Druckwandteil 3 und dem Verschlussteilträger 2 (Region B, siehe Fig. 2 und 5a,b) bei der Ausbildung der Druckkammer 4 geringeren Aufreisskräften ausgesetzt ist.

Eine solche Entlastung kann beispielsweise erreicht werden, wenn am Verschlussteil 1 oder am Verschlussteilträger 2 Hinterschnitte 13a,b oder hinterschnittartige Einformungen zum Zweck der Ausbildung von Gegendruckkammern vorhanden sind. Die Figuren 5a und b zeigen eine schematische Darstellung solcher Hinterschnitte 13a,b, entweder im Verschlussteilträger 2 (Fig. 5a) oder im Verschlussteil 1 (Fig. 5b). Zur Vereinfachung wurde in diesen Darstellungen der Haftkleber 6 und die Schutzschicht 7 weggelassen.

Eine Entlastung kann aber auch erreicht werden, wenn am Verschlussteil 1 oder am Verschlussteilträger 2 Dichtlippen 14a,b oder dichtlippenartige Ausformungen vorhanden sind. Die Figuren 6a und b zeigen eine schematische Darstellung solcher Dichtlippen 14a,b, entweder am Verschlussteilträger 2 (Fig. 6a) oder am Verschlussteil 1 (Fig. 6b). Zur Vereinfachung wurde in diesen Darstellungen der Haftkleber 6 und die Schutzschicht 7 weggelassen.

Im Falle einer Lösung gemäss den Figuren 5a und b können die Hinterschnitte 13a,b als Gegendruckkammern gesehen werden. Gegendruckkammern sind durch eine Öffnung mit der Druckkammer 4 verbunden , so dass die Gegendruckkammer im Gebrauch mit Blut füllbar ist, wobei die Gegendruckkammer mindestens eine Gegendruckfläche aufweist, die so orientiert ist, dass bei Befüllung der Gegendruckkammer mit Blut eine Druckkraftkomponente in eine Richtung erzeugt wird, die ein Zusammenpressen des Druckwandteils 3 und des Verschlussteilträgers 2 in der Region B bewirkt. Dies ist bei den Ausführungen gemäss den Figuren 5a und b der Fall, es kann aber auch mit anderen Formgebungen von Gegendruckkammern erreicht werden. Eine dieser Möglichkeiten bestünde beispielsweise darin, das Verschlussteil mit einer zentralen, sich nach innen aufweitenden Gegendruckkammer zu versehen (nicht dargestellt). Selbstverständlich sind hier auch viele Kombinationen von Kammerausformungen mit hinterschneidungs-artigen Ausformungen möglich. Im Falle einer Lösung gemäss den Figuren 6a und b werden die Dichtlippen 14a,b bei einer Anfüllung der Druckkammer 4 mit Blut gegen das Druckwandteil 3 gepresst, was eine Entlastung der Verbindung in der Region B oder der Regionen A und B bedeutet.

Schliesslich sind auch Kombinationen von Formgebungen nach den Prinzipien gemäss den Figuren 5a,b und 6a,b möglich. So könnte beispielsweise eine Formgebung gemäss der Figur 5b mit einer Formgebung gemäss der Figur 6a kombiniert werden.

### Bezugsziffernliste

- 1: Verschlussteil
- 2: Verschlussteilträger
- 3: Druckwandteil
- 4: Druckkammer
- 5: Ausnehmung
- 6: Haftkleber
- 7: Schutzschicht
- 8: Perforation
- 9: Ausläufer
- 10: Einkerbung
- 11a,b,c: Teilbereiche
- 12: Zentrum
- 13a,b: Hinterschnitte
- 14a,b: Dichtlippen

- A: Region chemischer Anbindung
- B: Region konventioneller Verbindung

## Patentansprüche

1. Vorrichtung zum Wiederverschliessen eines druckbeaufschlagbaren, komprimierbaren elastischen Gefässes, nach einer Punktion desselben, mittels des Mediums, welches den Druck in dem Gefäss erzeugt, mit
- einem erhabenen, vorzugsweise zylinderförmigen, tropfenförmigen oder halbkugelförmigen Verschlussteil (1) aus einem leicht durchdringbaren ersten Material mit hoher Rückstellkraft,
- einem Verschlussteilträger (2) oder Teilen eines Verschlussteilträgers (2) aus einem flexiblen und dehnbaren zweiten Material, und
- einem Druckwandteil (3) aus einem dünnen, flexiblen und dehnbaren dritten Material, das mit dem zweiten Material des Verschlussteilträgers (2) verbunden ist, wobei zwischen dem Verschlussteil (1) und dem Druckwandteil (3) eine Druckkammer (4) für das Medium ausgebildet oder ausbildbar ist, **dadurch gekennzeichnet, dass**
das Verschlussteil (1) in einer Ausnehmung (5) im Verschlussteilträger (2) unlösbar eingefügt ist.

2. Vorrichtung nach Patentanspruch 1, **dadurch gekennzeichnet, dass** sie für den Gebrauch am menschlichen oder tierischen Körper ausgebildet ist und auf dem Druckwandteil (3) ein hautfreundlicher Haftkleber (6) mit einer abziehbaren Schutzschicht (7) angebracht ist.

3. Vorrichtung nach einem der Patentansprüche 1 oder 2, **dadurch gekennzeichnet, dass** der Verschlussteilträger (2) und das Druckwandteil (3) aus gleichen oder gleichartigen Materialien bestehen, die eine gute Verbindbarkeit aufweisen zwecks Herstellung einer nicht aufreissbaren Verbindung zwischen dem Verschlussteilträger und dem Druckwandteil.

4. Vorrichtung nach Patentanspruch 3, **dadurch gekennzeichnet, dass** das zweite Material des Verschlussteilträger (2) oder Teile des Verschlussteilträgers (2) ein thermoplastisches Elastomer oder ein technischer Silikon ist.

5. Vorrichtung nach Patentanspruch 3, **dadurch gekennzeichnet, dass** das dritte Material des Druckwandteils (3) ein Polyurethanfilm ist.

6. Vorrichtung nach einem der Patentansprüche 1 bis 5, **dadurch gekennzeichnet, dass** das erste Material des Verschlussteils (1) einspritzbar ist und mit dem zweiten Material des Verschlussteilträgers (2) oder Teilen des Verschlussteilträgers (2) eine chemische Anbindung eingeht.

7. Vorrichtung nach Patentanspruch 6, **dadurch gekennzeichnet, dass** das erste Material des Verschlussteils (1) Silikon ist.

8. Vorrichtung nach einem der Patentansprüche 1 bis 7, **dadurch gekennzeichnet, dass** das Verschlussteil (1) und das Druckwandteil (3) transparent oder teilweise transparent sind.

9. Vorrichtung nach einem der Patentansprüche 1 bis 8, **dadurch gekennzeichnet, dass** das Verschlussteil (1) oder das Druckwandteil (3) im Bereich der Druckkammer (4) zwecks Vermeidung einer Adhäsion eine Aufrauhung aufweisen.

10. Vorrichtung nach Patentanspruch 8, **dadurch gekennzeichnet, dass** das Verschlussteil (1) verschiedenartig eingefärbte oder verschiedenartig getönte oder verschiedenartig oberflächig behandelte Schichten oder Teilbereiche (11a,b,c) zum Zweck der Vorgabe eines optimalen Einstichwinkels aufweist.

11. Vorrichtung nach Patentanspruch 8, **dadurch gekennzeichnet, dass** das Verschlussteil (1) vorstehende oder eingelassene Oberflächenstrukturen zum Zweck der Vorgabe eines optimalen Einstichwinkels aufweist.

12. Vorrichtung nach einem der Patentansprüche 1 bis 11, **dadurch gekennzeichnet, dass** am Verschlussteil (1) oder am Verschlussteilträger (2) Hinterschnitte (13a,b) oder hinterschnittartige Einformungen zum Zweck der Ausbildung von Gegendruckkammern vorhanden sind.

13. Vorrichtung nach einem der Patentansprüche 1 bis 11, **dadurch gekennzeichnet, dass** am Verschlussteil (1) oder am Verschlussteilträger (2) Dichtlippen (14a,b) oder dichtlippenartige Ausformungen angebracht sind.

14. Vorrichtung nach Patentanspruch 1, **dadurch gekennzeichnet, dass** sie für den Gebrauch von Probenahmen aus druckbeaufschlagten durchstechbaren Gefässen oder Behältnissen unter Labor-, Praxis- oder Feldbedingungen ausgebildet ist und auf dem Druckwandteil (3) ein Haftkleber mit einer abziehbaren Schutzschicht angebracht ist.
